# EUROPEAN PATENT APPLICATION

(11) **EP 4 289 815 A1**
(43) Date of publication of application: **13.12.2023**
(21) Application number: 22305846.2
(22) Date of filing: 10.06.2022
(51) Int. Cl.: C07C 225/20, A61K 31/135, A61P 25/24, A61P 29/02

(54) **CRYSTALLINE FORMS OF NORKETAMINE**

(71) Applicant: PCAS, 69134 Ecully Cedex (FR)
(72) Inventor: LEROUDIER, Julien, 78200 Buchelay (FR); SOUILLARD, Olivier, 27600 Gaillon (FR)
(74) Representative: Regimbeau

(57) **Abstract**

The present disclosure relates to crystalline forms of norketamine, in particular norketamine hydrochloride, processes for the preparation and uses thereof.

## Description

### FIELD OF THE INVENTION

The invention relates to crystalline forms of norketamine, in particular norketamine hydrochloride, processes for preparation and uses thereof.

### BACKGROUND OF THE INVENTION

Ketamine, also known as (R,S)-2-(2-chlorophenyl)-2-(methylamino)cyclohexanone, has been used as an anesthetic for human and veterinary uses for over fifty years. Ketamine has also been used for treating other conditions, e.g. to alleviate pain or depression.

One of the major active metabolic products of ketamine is norketamine ((R, S)-2-(2-chlorophenyl)-2-amino-cyclohexanone). *In vivo,* ketamine is converted through demethylation into norketamine at rates which depend on the route of administration. Like ketamine, norketamine acts as a noncompetitive N-methyl-D-aspartate (NMDA) receptor antagonist. Norketamine has also been reported to be useful for treating pain (WO2004045601) or to treat depressive symptom (WO2018079693).

Polymorphic forms are of great interest to the pharmaceutical industry and especially to those involved in the development of manufacturing processes and in the development of suitable dosage forms. Polymorphic forms are different crystalline forms of the same compound. The various crystalline forms of a compound differ not only in appearance and hardness, but also in numerous other physicochemical properties (stability, filterability, solubility, hygroscopicity, melting point, solid density and flowability). Differences in stability, filterability, solubility, hygroscopicity, melting point, solid density and flowability can have a strong influence on the quality and effectiveness of the drug. It is not yet possible to predict the occurrence and number of crystalline forms including their physicochemical properties. Above all, the thermodynamic stability cannot be determined in advance.

For pharmaceutical development and commercialization, there is a need to identify a solid form of norketamine hydrochloride that can be readily manufactured, processed and formulated. Consequently, there is a need to identify a solid form of norketamine hydrochloride having desirable physicochemical and manufacturing properties, i.e. a solid form that can be readily manufactured at low cost in high purity and in large quantities and that can be readily formulated.

### SUMMARY OF THE INVENTION

The invention relates to a crystalline form of (R,S)-2-amino-2-(2-chlorophenyl)cyclohexan-1-one hydrochloride.

In an aspect, it relates to a crystalline form of (R,S)-2-amino-2-(2-chlorophenyl)cyclohexan-1-one hydrochloride wherein said crystalline form is characterized by a powder X-ray diffraction (PXRD) pattern comprising peaks at about 9.3 and 14.4 degrees 2θ (+/- 0.2 degrees 2θ), preferably at about 9.3, 11.8, 13.5, 14.4, 15.6, 18.1, 20.8, 22.15, 25.7 and 26.9 degrees 2θ (+/- 0.2 degrees 2θ) and to a method for making thereof.

In another aspect, it relates to a crystalline form of (R,S)-2-amino-2-(2-chlorophenyl)cyclohexan-1-one hydrochloride wherein said crystalline form is characterized by a powder X-ray diffraction (PXRD) pattern comprising peaks at about 16.1 and 23.1 degrees 2θ (+/- 0.2 degrees 2θ), preferably at about 12.0, 13.4, 15.6, 16.1, 18.4, 20.9, 23.1, 24.2, 25.3 and 33.8 degrees 2θ (+/- 0.2 degrees 2θ) and to a method for making thereof.

The invention also relates to a pharmaceutical composition comprising the herein disclosed crystalline form(s), in particular for use in treating pain or treating depressive symptom.

Further aspects of the invention are as disclosed herein and in the claims.

### FIGURES

Figure 1: PXRD profile of crystalline form of (R,S)-2-amino-2-(2-chlorophenyl)cyclohexan-1-one hydrochloride prepared in accordance with example 1.
Figure 2: DSC profile of crystalline form of (R,S)-2-amino-2-(2-chlorophenyl)cyclohexan-1-one hydrochloride prepared in accordance with example 1.
Figure 3: PXRD profile of crystalline form of (R,S)-2-amino-2-(2-chlorophenyl)cyclohexan-1-one hydrochloride prepared in accordance with example 4.
Figure 4: TGA/DTA curves of crystalline form of (R,S)-2-amino-2-(2-chlorophenyl)cyclohexan-1-one hydrochloride prepared in accordance with example 4.
Figure 5: DSC profile of crystalline form of (R,S)-2-amino-2-(2-chlorophenyl)cyclohexan-1-one hydrochloride prepared in accordance with example 4.

### DEFINITIONS

"Norketamine" or "norketamine base" as used herein designate a racemic mixture of 2-amino-2-(2-chlorophenyl)cyclohexan-1-one: (R, S)-2-(2-chlorophenyl)-2-amino-cyclohexanone.

"Norketamine hydrochloride" as used herein designates the hydrochloride salt of 2-amino-2-(2-chlorophenyl)cyclohexan-1-one (racemic mixture): (R, S)-2-(2-chlorophenyl)-2-amino-cyclohexanone hydrochloride.

### DESCRIPTION OF THE INVENTION

The inventors have identified novel crystalline forms of a pharmaceutically acceptable salt of (R,S)-2-amino-2-(2-chlorophenyl)cyclohexan-1-one. More particularly, the inventors have identified crystalline forms of (R, S)-2-amino-2-(2-chlorophenyl)cyclohexan-1-one hydrochloride having desirable properties such as high crystallinity, high purity and favorable physical stability, chemical stability, dissolution and mechanical properties. In particular, the identified crystalline forms appear to be the most stable under conditions encountered during formulation process (tableting) and storage, in particular the most stable prior to or after curing.

Therefore, the present invention relates to a crystalline form of (R,S)-2-amino-2-(2-chlorophenyl)cyclohexan-1-one hydrochloride.

In one embodiment, the invention relates to a crystalline form of (R,S)-2-amino-2-(2-chlorophenyl)cyclohexan-1-one hydrochloride that is characterized by a powder X-ray diffraction (PXRD) pattern comprising peaks at about 9.3 and 14.4degrees 2θ (+/- 0.2 degrees 2θ), preferably at about 9.3, 11.8, 13.5, 14.4, 15.6, 18.1, 20.8, 22.2, 25.7 and 26.9 degrees 2θ (+/- 0.2 degrees 2θ). This crystalline form is referred herein as pattern 1.

Pattern 1 is further characterized by its differential scanning calorimetry thermogram. Pattern 1 has an endotherm in the range of 230 to 250 °C, preferably in the range of 235 to 242°C, more preferably with onset value at about 238 °C.

Pattern 1 is an anhydrous form of (R,S)-2-amino-2-(2-chlorophenyl)cyclohexan-1-one hydrochloride. Said form has been found to be the most stable form after formulation.

In some embodiments, the crystalline form of the present invention comprises at least 50 wt% of pattern 1, i.e., at least 50 wt% of a crystalline form of (R,S)-2-amino-2-(2-chlorophenyl)cyclohexan-1-one hydrochloride that is characterized by a powder X-ray diffraction (PXRD) pattern comprising peaks at about 9.3 and 14.4 degrees 2θ (+/- 0.2 degrees 2θ), preferably at about 9.3, 11.8, 13.5, 14.4, 15.6, 18.1, 20.8, 22.2, 25.7 and 26.9 degrees 2θ (+/- 0.2 degrees 2θ) and/or an endotherm in the range of 230 to 250 °C, preferably in the range of 235 to 242°C, more preferably with onset value at about 238 °C. In some embodiments, the crystalline form of the present invention comprises at least 60 wt%, or at least 70 wt% or at least 80wt% or at least 90wt% or at least 95 wt% of pattern 1.

In another embodiment, the invention relates to a crystalline form of (R,S)-2-amino-2-(2-chlorophenyl)cyclohexan-1-one hydrochloride that is characterized by a powder X-ray diffraction (PXRD) pattern comprising peaks at about 16.1 and 23.1 degrees 2θ (+/- 0.2 degrees 2θ), preferably at about 12.1, 13.4, 15.6, 16.1, 18.4, 20.9, 23.1, 24.2, 25.3 and 33.8 degrees 2θ (+/- 0.2 degrees 2θ). Said crystalline form is referred herein as pattern 2.

Pattern 2 is further characterized by its differential scanning calorimetry thermogram. Pattern 2 has an endotherm in the range of 85 to 95 °C, preferably with onset value at about 90°C, optionally a second endotherm in the range of 95 to 105°C, preferably with onset value at about 100°C, and optionally a third endotherm in the range of 235 to 242°C, more preferably with onset value at about 239 °C.

Pattern 2 is an hydrate form of (R,S)-2-amino-2-(2-chlorophenyl)cyclohexan-1-one hydrochloride with about 1.8 equivalents of water.

Pattern 2 has been found to be stable over a large range of temperature and humidity. This form is then particularly advantageous from a formulation standpoint. It is stable upon storage, offers good handling properties and remains stable under most of the conditions encountered during tableting.

In some embodiments, the crystalline form of the present invention comprises at least 50 wt% of pattern 2, i.e., at least 50 wt% of a crystalline form of (R,S)-2-amino-2-(2-chlorophenyl)cyclohexan-1-one hydrochloride that is characterized by a powder X-ray diffraction (PXRD) pattern comprising peaks at about 16.1 and 23.1 degrees 2θ (+/- 0.2 degrees 2θ), preferably at about 12.1, 13.4, 15.6, 16.1, 18.4, 20.9, 23.1, 24.2, 25.3 and 33.8 degrees 2θ (+/- 0.2 degrees 2θ) and/or an endotherm in the range of 85 to 95 °C, preferably at 90°C, optionally a second endotherm in the range of 95 to 105°C, preferably at 100°C, and optionally a third endotherm in the range of 235 to 242°C, more preferably at 239 °C. In some embodiments, the crystalline form of the present invention comprises at least 60 wt%, or at least 70 wt% or at least 80wt% or at least 90wt% or at least 95 wt% of pattern 2.

The present invention also relates to a method for preparing a crystalline form of (R,S)-2-amino-2-(2-chlorophenyl)cyclohexan-1-one hydrochloride comprising the step of dissolving (R,S)-2-amino-2-(2-chlorophenyl)cyclohexan-1-one hydrochloride in an organic solvent typically selected from the group consisting of alcohols (e.g. ethanol, methanol) and DMSO at a temperature that would allow to conduct a polish filtration (typically about 20 to 80°C). The solution is then cooled down (typically to about 0-10°C) to recrystallize Pattern 1 material in a controlled way (cooling rate is typically between 5 and 20°C/h). The suspension is either centrifuged or filtered prior to be dried. The latter allows to produce Pattern 1 material meeting the International Council for Harmonisation of Technical Requirements for Pharmaceuticals for Human Use (ICH) limits in terms of residual solvents. The solid produced requires to be stored at a temperature and relative humidity where Pattern 1 is the most stable form (low relative humidity typically below 30%).

The present invention also relates to a method for preparing a crystalline form of (R,S)-2-amino-2-(2-chlorophenyl)cyclohexan-1-one hydrochloride comprising the step of dissolving (R,S)-2-amino-2-(2-chlorophenyl)cyclohexan-1-one hydrochloride in a solvent mixture, typically comprising ethers, ketones or ester mixed with water, at a temperature that would allow to conduct a polish filtration (typically about 20 to 80°C). The solution is then cooled down (typically to about 0-10°C) to recrystallize Pattern 2 material in a controlled way (cooling rate is typically between 5 and 20°C/h). The suspension is either centrifuged or filtered prior to be dried. The latter allows to produce Pattern 2 material meeting the ICH limits in terms of residual solvents (organic), typically using a pressure filter dryer with a controlled humidity and temperature (typically from room temperature to 40°C and from 50 to 90% relative humidity). The solid produced requires to be stored at a temperature and relative humidity where Pattern 2 is the most stable form.

The crystalline forms as described herein can be administered alone or as a formulation in association with one or more pharmaceutically acceptable excipients. Thus, the present invention also relates to a pharmaceutical composition comprising any of the above disclosed crystalline forms, typically a therapeutically effective amount of said crystalline forms, and at least one pharmaceutically acceptable excipient. The choice of excipient will to a large extent depend on factors such as the particular mode of administration, the effect of the excipient on solubility and stability, and the nature of the dosage form.

The terms "pharmaceutically acceptable excipient" refers to a material (or materials) that may be included with a particular active pharmaceutical agent to form a pharmaceutical composition, and may be solid or liquid. Pharmaceutically acceptable excipients include, but are not limited to surfactants, lubricants, binders, fillers, compression aids, disintegrants, water-soluble polymers, inorganic salts, preservatives, antioxidants, coloring agents, sweetening agents, souring agents, bubbling agents and flavorings.

The form of the pharmaceutical composition preferably comprises solid state composition (e.g., tablet, pill, capsule containing particulates or powders, pellet, granule, powder etc.). The pharmaceutical composition may be compressed directly or by roller to form tablets.

The crystalline forms or the pharmaceutical composition as described herein may be used for treating pain or treating depressive symptom. Hence, the invention also relates to a method for treating pain or depressive symptom comprising administering to a subject in need thereof of a therapeutically effective amount of any of the above disclosed crystalline forms or compositions.

Embodiments of the present invention will now be described by way of the following examples which are provided for illustrative purposes only, and not intended to limit the scope of the disclosure.

### EXAMPLES

### Example 1: Synthesis of crystalline (R,S)-2-amino-2-(2-chlorophenyl)cyclohexan-1-one hydrochloride (pattern 1)

Crystalline (R,S)-2-amino-2-(2-chlorophenyl)cyclohexan-1-one hydrochloride was produced by slurrying amorphous material in MTBE solvent for one week at room temperature under stirring. The solid was isolated.

### Example 2: PXRD analysis of crystalline (R,S)-2-amino-2-(2-chlorophenyl)cyclohexan-1-one hydrochloride (pattern 1)

A sample of crystalline (R,S)-2-amino-2-(2-chlorophenyl)cyclohexan-1-one hydrochloride prepared in accordance with example 1 was analyzed by PXRD. Powder X-ray diffraction data were collected on Empyrean diffractometer from Malvern-Panalytical equipped with a Cu radiation source.

Details regarding the equipment and measurement parameters used for the XRPD analysis are described below:

| | |
|---|---|
| Source: | Cu, Kα; 45 kV, 40 mA |
| Detector: | PlXcel 1D |
| Divergence slit: | Fixed, 10mm |
| Spinner: | 1s |
| Step size: | 0.013° (2θ) |
| Measured time per step: | 29.07s |
| Scan range: | 2-40° (2θ) |
| Configuration : | Transmission |

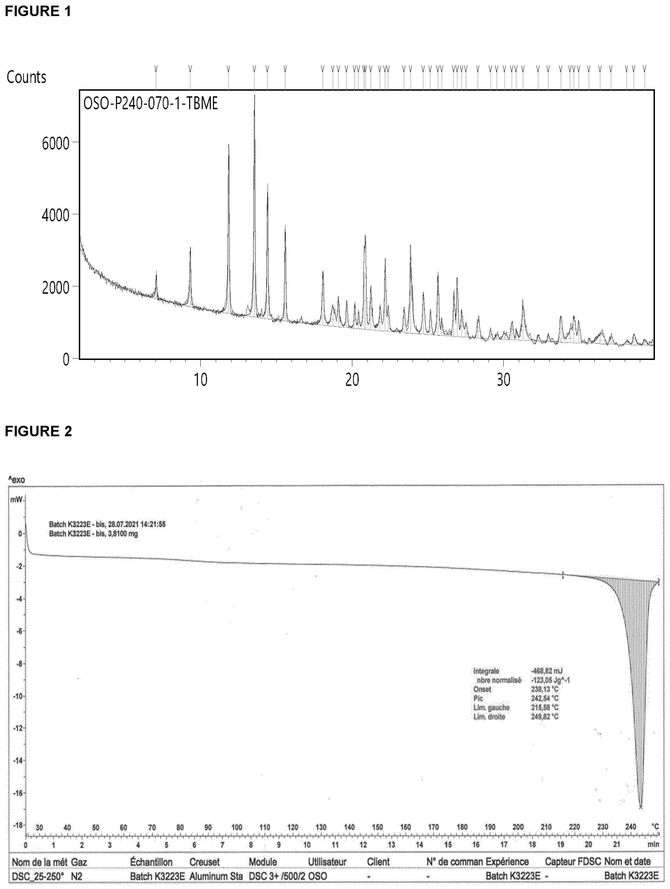

The XRPD pattern for the sample is provided in Figure 1. A typical error of ± 0.2° 2-theta in peak positions applies to this data.

Peak list is provided in Table 1 below.

**Table 1: peak list of crystalline 2-amino-2-(2-chlorophenyl)cyclohexan-1-one hydrochloride (pattern 1)**

| **Peak number** | **Peak position (°2θ)** | **Relative intensity (%)** |
|---|---|---|
| **1** | 9.3 | 26 |
| **2** | 11.8 | 77 |
| **3** | 13.5 | 100 |
| **4** | 14.4 | 63 |
| **5** | 15.6 | 44 |
| **6** | 18.1 | 27 |
| **7** | 20.8 | 38 |
| **8** | 22.2 | 35 |
| **9** | 25.7 | 33 |
| **10** | 26.9 | 33 |

### Example 3: DSC analysis of crystalline (R,S)-2-amino-2-(2-chlorophenyl)cyclohexan-1-one hydrochloride (pattern 1)

Thermal behaviour of a sample of crystalline (R,S)-2-amino-2-(2-chlorophenyl)cyclohexan-1-one hydrochloride prepared in accordance with example 1 was characterized by Differential Scanning calorimetry (DSC) analysis using a DSC3+ from Mettler Toledo (analysis from 25-250°C at 10°C/min). The DSC profile of the sample is provided in figure 2.

An endotherm was observed at around 238°C (Onset temperature) suggesting the melting of the material (anhydrous form).

### Example 4: Synthesis of crystalline (R,S)-2-amino-2-(2-chlorophenyl)cyclohexan-1-one hydrochloride (pattern 2)

Crystalline (R,S)-2-amino-2-(2-chlorophenyl)cyclohexan-1-one hydrochloride was produced by stressing amorphous (R,S)-2-amino-2-(2-chlorophenyl)cyclohexan-1-one hydrochloride at ambient temperature and at 75% relative humidity for 3 days prior to expose the solid for 4 days at 99% relative humidity.

### Example 5: PXRD analysis of crystalline (R,S)-2-amino-2-(2-chlorophenyl)cyclohexan-1-one hydrochloride (pattern 2)

A sample of crystalline (R,S)-2-amino-2-(2-chlorophenyl)cyclohexan-1-one hydrochloride prepared in accordance with example 4 was analyzed by PXRD. Powder X-ray diffraction data were collected on Empyrean diffractometer from Malvern-Panalytical equipped with a Cu radiation source.

Details regarding the equipment and measurement parameters used for the XRPD analysis are described below:

| | |
|---|---|
| Source: | Cu, Kα; 45 kV, 40 mA |
| Detector: | PlXcel 1D |
| Divergence slit: | Fixed, 10mm |
| Spinner: | 1s |
| Step size: | 0.007° (2θ) |
| Measured time per step: | 46.92s |
| Scan range: | 2-40° (2θ) |
| Configuration | Transmission |

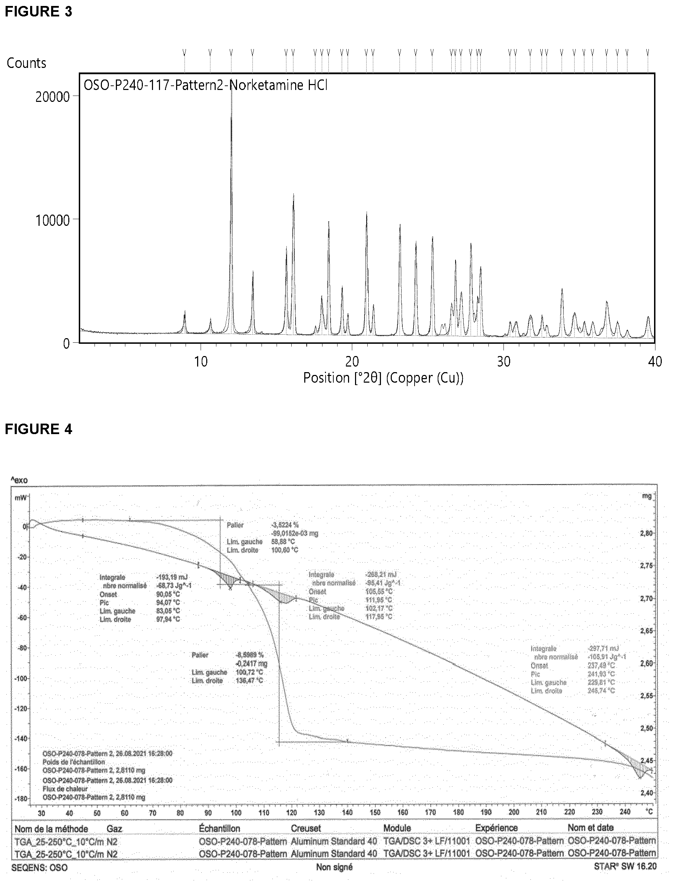

The PXRD profile for the sample is provided in Figure 3. A typical error of ± 0.2° 2-theta in peak positions applies to this data.

Peak list is provided in Table 2 below.

**Table 2: peak list of crystalline 2-amino-2-(2-chlorophenyl)cyclohexan-1-one hydrochloride (pattern 2)**

| **Peak number** | **Peak position (°2θ)** | **Relative intensity (%)** |
|---|---|---|
| **1** | 12.0 | 100 |
| **2** | 13.4 | 26 |
| **3** | 15.6 | 39 |
| **4** | 16.1 | 59 |
| **5** | 18.4 | 49 |
| **6** | 20.9 | 58 |
| **7** | 23.1 | 53 |
| **8** | 24.2 | 28 |
| **9** | 25.3 | 48 |
| **10** | 33.8 | 25 |

### Example 6: TGA/DTA and DSC analysis of crystalline (R,S)-2-amino-2-(2-chlorophenyl)cyclohexan-1-one hydrochloride (pattern 2)

Thermal behaviour of a sample of crystalline (R,S)-2-amino-2-(2-chlorophenyl)cyclohexan-1-one hydrochloride prepared in accordance with example 4 was characterized by Thermogravimetric Analysis/ Differential Thermal Analysis (TGA/DTA) analysis and by DSC analysis using a TGA/DSC 3+ and DSC3+ from Mettler Toledo.

The TGA/DTA curves of the sample is provided in figure 4.

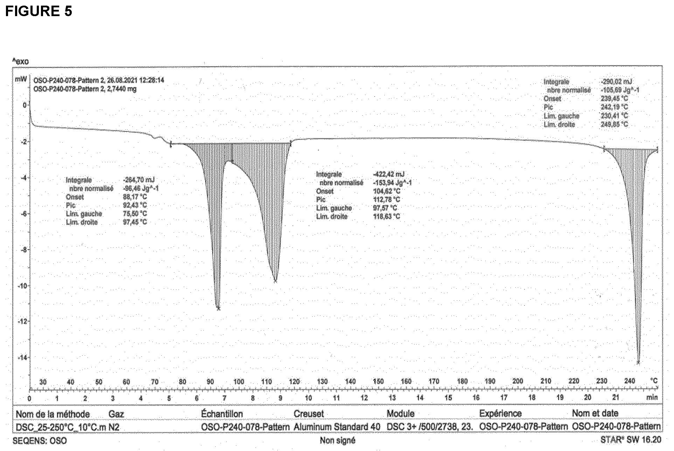

The DSC profile of the sample is provided in figure 5.

Results exhibited the presence of 3 endotherms:
- The 1^{st} endotherm at ~90°C associated with a weight loss of ~3.5%w/w (possibly a first dehydration of pattern 2);
- This was followed by another endotherm at ~100°C associated with a weight loss of ~8.5% w/w possibly a further dehydration towards pattern 1;
- A final endotherm at ~239°C was observed likely corresponding to the melting concomitant to degradation of pattern 1.

### Example 7: Stability testing (pattern 1 vs. pattern 2)

For each condition, a sample of the form of interest was placed into a controlled humidity and temperature chamber for up to 7 days. It should be noted that, for the condition at 70°C, only the temperature was controlled. At the end of this stressing test, all solids were analyzed by XRPD analysis to verify the crystalline form and compared to the input material.

**Table 3: Stability testing between Patterns 1 vs Pattern 2**

| **Input material** | **Conditions** | **Results** |
|---|---|---|
| Pattern 1 | 25°C / 60%RH | Pattern 2 |
| | 30°C / 65%RH | |
| | 30°C / 75%RH | |
| | 40°C / 75%RH | |
| | After 2 hours at 70°C | Pattern 1 |
| Pattern 2 | 25°C / 60%RH | Pattern 2 |
| | 30°C / 65%RH | |
| | 30°C / 70%RH | |
| | 40°C / 75%RH | |
| | After 2 hours at 70°C | Pattern 1 |

**Table 4: Water activities experiments of Norketamine HCl**

| **Solvents** | **T(°C)** | **A_{w}** | **Results** |
|---|---|---|---|
| Ethanol/water | 25 | ~0,1 | Pattern 1 |
| | | ~0,2 | Pattern 1 |
| | | ~0,3 | Pattern 1 |
| | | ~0,5 | Pattern 2 |
| Ethanol / water | 25 | ~0,7 | Pattern 2 |
| Acetone / water | | ~0,9 | Pattern 2 |

## Claims

1. A crystalline form of (R,S)-2-amino-2-(2-chlorophenyl)cyclohexan-1-one hydrochloride.

2. The crystalline form of (R,S)-2-amino-2-(2-chlorophenyl)cyclohexan-1-one hydrochloride according to claim 1 wherein said crystalline form is **characterized by** a powder X-ray diffraction (PXRD) pattern comprising peaks at about 9.3 and 14.4 degrees 2θ (+/- 0.2 degrees 2θ), preferably at about 9.3, 11.8, 13.5, 14.4, 15.6, 18.1, 20.8, 22.15, 25.7 and 26.9 degrees 2θ (+/- 0.2 degrees 2θ).

3. The crystalline form of (R,S)-2-amino-2-(2-chlorophenyl)cyclohexan-1-one hydrochloride according to claim 2, wherein the differential scanning calorimetry thermogram has an endotherm in the range of 230 to 250 °C, preferably in the range of 235 to 242°C, more preferably with onset value at 238 °C.

4. The crystalline form of (R,S)-2-amino-2-(2-chlorophenyl)cyclohexan-1-one hydrochloride according to claim 1, wherein at least 50% of said (R,S)-2-amino-2-(2-chlorophenyl)cyclohexan-1-one hydrochloride is present in the form of the crystalline form according to claim 2 or 3, preferably at least 60%, or 70% or 80% or 90% or 95% of said (R,S)-2-amino-2-(2-chlorophenyl)cyclohexan-1-one hydrochloride is present in the form of the crystalline form according to claim 2 or 3.

5. The crystalline form of (R,S)-2-amino-2-(2-chlorophenyl)cyclohexan-1-one hydrochloride according to claim 1 wherein said crystalline form is **characterized by** a powder X-ray diffraction (PXRD) pattern comprising peaks at about 16.1 and 23.1 degrees 2θ (+/- 0.2 degrees 2θ), preferably at about 12.0, 13.4, 15.6, 16.1, 18.4, 20.9, 23.1, 24.2, 25.3 and 33.8 degrees 2θ (+/- 0.2 degrees 2θ).

6. The crystalline form of (R,S)-2-amino-2-(2-chlorophenyl)cyclohexan-1-one hydrochloride according to claim 5, wherein the differential scanning calorimetry thermogram has an endotherm in the range of 85 to 95 °C, preferably at 90°C, optionally a second endotherm in the range of 95 to 105°C, preferably at 100°C, and optionally a third endotherm in the range of 235 to 242°C, more preferably with onset value at 239 °C.

7. The crystalline form of (R,S)-2-amino-2-(2-chlorophenyl)cyclohexan-1-one hydrochloride according to claim 1, wherein at least 50% of said (R,S)-2-amino-2-(2-chlorophenyl)cyclohexan-1-one hydrochloride is present in the form of the crystalline form according to claim 5 or 6, preferably at least 60%, or 70% or 80% or 90% or 95% of said (R,S)-2-amino-2-(2-chlorophenyl)cyclohexan-1-one hydrochloride is present in the form of the crystalline form according to claim 5 or 6.

8. A method for making the crystalline form according to any one of claims 2 to 4, said method comprising the following steps:
dissolving (R,S)-2-amino-2-(2-chlorophenyl)cyclohexan-1-one hydrochloride in an organic solvent selected from the group consisting of alcohols and DMSO at a temperature that allows to conduct a polish filtration (typically about 20 to 80°C) and cooling down the solution to recrystallize and obtain the recited form.

9. A method for making the crystalline form according to any one of claims 5 to 7, said method comprising the following steps:
dissolving (R,S)-2-amino-2-(2-chlorophenyl)cyclohexan-1-one hydrochloride in a solvent mixture comprising ethers, ketones or ester mixed with water, at a temperature that allows to conduct a polish filtration (typically about 20 to 80°C) and cooling down the solution to recrystalliz;e and obtain the recited form.

10. A pharmaceutical composition comprising the crystalline form of any one of claims 1 to 7 and at least one pharmaceutically acceptable excipient.

11. The crystalline form of any one of claims 1 to 7 or the pharmaceutical composition of claim 10 for use in treating pain or treating depressive symptom.
